# FASCICULE DE BREVET EUROPEEN

(11) **EP 4 046 641 B1**
(45) Date de publication et mention de la délivrance du brevet: **19.08.2026**
(21) Numéro de dépôt: 22168015.0
(22) Date de dépôt: 25.10.2017
(51) Int. Cl.: A61K 35/33, A61K 35/38, A61P 19/02

(54) **COMPOSITION POUR LE TRAITEMENT D'UNE AFFECTION DE L APPAREIL LOCOMOTEUR CHEZ UN CHIEN**
ZUSAMMENSETZUNG ZUR BEHANDLUNG EINER ERKRANKUNG DES BEWEGUNGSAPPARATS EINES HUNDES
COMPOSITION FOR THE TREATMENT OF A DISORDER OF THE MUSCULOSKELETAL SYSTEM IN A DOG

(30) Priorité: 27.10.2016 FR 1660464
(43) Date de publication de la demande: 24.08.2022
(62) Numéro(s) de document de la (des) demande(s) initiale(s) en application de l'article 76 CBE: 17801349.6 / 3 532 080
(73) Titulaire: Scarcell Therapeutics, 75006 Paris (FR)
(72) Inventeur: LAFONT, Antoine, 75007 Paris (FR); COULOMB, Bernard, 91430 Igny (FR)
(74) Mandataire: Vial, Lionel

(56) Documents cités:
- WO-A1-2004/022078
- WO-A1-2011/070305
- FR-A1- 2 980 710
- NIELS MENSING ET AL: "Isolation and characterization of multipotent mesenchymal stromal cells from the gingiva and the periodontal ligament of the horse", BMC VETERINARY RESEARCH, BIOMED CENTRAL, LONDON, GB, vol. 7, no. 1, 2 August 2011 (2011-08-02), pages 42, XP021105887, ISSN: 1746-6148, DOI: 10.1186/1746-6148-7-42

## Description

### Domaine de l'invention

La présente invention concerne une composition utile pour la prévention ou le traitement d'une affection de l'appareil locomoteur .

### Arrière-plan technique

En France, le nombre total de chevaux est d'environ 350 000 dont approximativement 28 000 chevaux de course. 20 à 30% d'entre eux présentent des tendinopathies. Cela représente environ 8000 cas par an, si l'on ne considère que les courses hippiques.

Aucune méthode de traitement classique, à base d'anti-inflammatoires, n'apporte de réels bénéfices par rapport à une cicatrisation naturelle sur le long terme, laquelle implique un repos de 6 mois à un an. De plus, ces traitements sont essentiellement symptomatiques et ne guérissent pas le tendon.

Il a donc été proposé d'utiliser des cellules souches, notamment de moelle osseuse, de cordon ombilical ou de tissu adipeux, afin d'obtenir une cicatrisation tendineuse se rapprochant du tendon d'origine.

Les cellules de tissu adipeux semblent cependant se différencier moins bien que les cellules de moelle osseuse. En outre, fiabilité des cellules souches issues de cordon ombilicale n'est pas véritablement établie.

Ainsi, ce sont les cellules souches mésenchymateuses de moelle osseuse qui font actuellement figure de traitement de référence de la tendinite du cheval (Godwin et al. (2012) Equine Veterinary Journal 44:25-32).

Toutefois, le prélèvement de moelle osseuse est une procédure relativement lourde. De plus, les cellules souches mésenchymateuses de moelle osseuse ont été essentiellement validées pour une utilisation autologue, c'est-à-dire que les cellules proviennent de l'animal auquel elles sont administrées, ce qui renchérit le coût de cette méthode.

Il serait donc intéressant de disposer de traitements alternatifs, plus simples et moins coûteux à mettre en œuvre.

### Résumé de l'invention

La présente invention découle de la mise en évidence inattendue, par les inventeurs, que l'administration d'une composition à base de fibroblastes gingivaux hétérologues à des chevaux souffrant de tendinites ou d'ostéochondrose permettait de traiter ces affections.

Avantageusement, les fibroblastes gingivaux sont facilement prélevés et préparés. En outre, ils sont efficaces même dans le cadre d'une utilisation hétérologue.

### L'invention est telle que définie dans les revendications.

### Description détaillée de l'invention

A titre préliminaire, on rappellera que le terme « comprenant» signifie «incluant», «contenant» ou «englobant», c'est-à-dire que lorsqu'un objet «comprend» un élément ou plusieurs éléments, d'autres éléments que ceux mentionnés peuvent également être compris dans l'objet. A contrario, l'expression « consistant en » signifie « constitué de», c'est-à-dire que lorsqu'un objet « consiste en » un élément ou plusieurs éléments, l'objet ne peut pas comprendre d'autres éléments que ceux mentionnés.

### Affection de l'appareil locomoteur

Comme on l'entend ici une affection de l'appareil locomoteur désigne notamment une affection ostéo-articulaire ou musculo-squelettique, notamment d'un membre.

Les affections de l'appareil locomoteur du cheval sont notamment décrites dans « Maladie des Chevaux » (1994) Institut du cheval, France Agricole Editions.

L'affection de l'appareil locomoteur selon l'invention est une tendinite.

Plus préférablement, l'affection de l'appareil locomoteur selon l'invention est une tendinite d'un tendon fléchisseur, notamment superficiel ou profond des phalanges, ou d'un tendon extenseur, notamment latéral des phalanges ou antérieur des phalanges, en particulier chez un cheval.

Comme on l'entend ici le tendon fléchisseur superficiel est également nommé tendon perforé et le tendon profond des phalanges est également nommé tendon perforant.

Alternativement, l'affection de l'appareil locomoteur selon l'invention est une ostéochondrose ou une arthrose .

Comme on l'entend ici, l'arthrose est synonyme d'ostéoarthrite.

De préférence, l'ostéochondrose ou l'arthrose est associée à des nodules osseux, à une ostéochondrite dissécante ou à un kyste osseux

### Individu

L'individu selon l'invention est un L'individu selon l'invention peut être mâle ou femelle.

### Composition

Comme on l'entend ici, l'expression « cellules buccales » est équivalente à « cellules de la cavité orale » ou « cellules de la muqueuse orale ».

Les cellules buccales selon l'invention sont des cellules de la gencive.

De préférence, la composition selon l'invention est issue de la mise en culture d'un prélèvement de gencive.

La composition selon l'invention comprend ou est constituée de fibroblastes gingivaux. On parle alors de composition de fibroblastes gingivaux. La composition selon l'invention peut notamment être une culture, éventuellement concentrée, de fibroblastes gingivaux ou une suspension de fibroblastes gingivaux.

Les procédures pour prélever, cultiver, et conserver les fibroblastes gingivaux sont bien connues de l'homme du métier et sont notamment décrites dans Naveau et al. (2006) J. Periodontol. 77:238-47 et dans Gogly et al. (2007) Arterioscler. Thromb. Vasc. Biol. 27:1984-90, ainsi que dans les Exemples ci-après. En particulier, les fibroblastes gingivaux peuvent être obtenus par la mise en culture d'un prélèvement ou d'une biopsie de gencive, éventuellement après digestion enzymatique du prélèvement ou de la biopsie pour libérer les fibroblastes gingivaux qui la constituent. Ainsi, les cellules issues de la mise en culture d'un prélèvement ou d'une biopsie de gencive sont essentiellement des fibroblastes gingivaux.

Dans un mode de réalisation de l'invention, les fibroblastes gingivaux selon l'invention comprennent des cellules souches mésenchymateuses. Comme l'homme du métier le comprend bien les cellules souches mésenchymateuses sont alors des cellules souches mésenchymateuses buccales, c'est-à-dire de la cavité orale ou de la muqueuse orale, notamment de la muqueuse de la joue, de la langue, du palais, de la muqueuse labiale, de la muqueuse sublinguale, ou de la gencive.

Dans un autre mode de réalisation de l'invention, les fibroblastes gingivaux selon l'invention ne comprennent pas, ou essentiellement pas, de cellules souches mésenchymateuses.

Avantageusement, les fibroblastes gingivaux sont aisément prélevables et cultivables. En outre, les fibroblastes gingivaux présentent une vitesse de croissance importante.

Comme l'homme du métier le comprend bien, la composition selon l'invention comprend les fibroblastes gingivaux selon l'invention en une quantité prophylactiquement ou thérapeutiquement efficace. De préférence, la composition selon l'invention comprend de 5 millions à 40 millions de fibroblastes gingivaux, selon l'invention, plus préférablement de 10 millions à 30 millions de fibroblastes gingivaux selon l'invention, encore plus préférablement de 15 à 25 millions de fibroblastes gingivaux selon l'invention, et le plus préférablement environ 20 millions de fibroblastes gingivaux selon l'invention. La quantification des fibroblastes gingivaux selon l'invention peut être effectuée par toute méthode de numération des cellules de mammifères connues de l'homme du métier et notamment à l'aide d'un automate compteur de cellules.

De préférences, les fibroblastes gingivaux, selon l'invention sont hétérologues, c'est-à-dire qu'ils proviennent d'un autre individu, notamment d'un autre cheval, que celui chez lequel on utilise la composition. Comme l'homme du métier le comprendra bien, l'individu chez lequel on prélève les cellules et l'individu auquel on administre les cellules sont préférablement de la même espèce.

De préférence également, les fibroblastes gingivaux selon l'invention sont autologues, c'est-à-dire qu'ils proviennent du même individu, notamment du même cheval, que celui chez lequel on utilise la composition.

De préférence, la composition selon l'invention est administrée à proximité ou au niveau d'un site corporel à traiter, plus préférablement dans la lésion à traiter. L'administration peut être effectuée par n'importe quelle méthode connue de l'homme du métier compatible avec l'administration de cellules. On préfèrera cependant que la composition soit injectée, en particulier dans la lésion à traiter. De préférence, la composition selon l'invention est ainsi injectée au niveau d'une tendinite à traiter, plus préférablement dans la lésion tendineuse, notamment par échoguidage, ou dans ou au niveau d'une articulation présentant une ostéochondrose ou une arthrose.

L'invention sera davantage explicitée de manière non limitative par la Figure et les Exemples qui suivent.

### Description de la figure

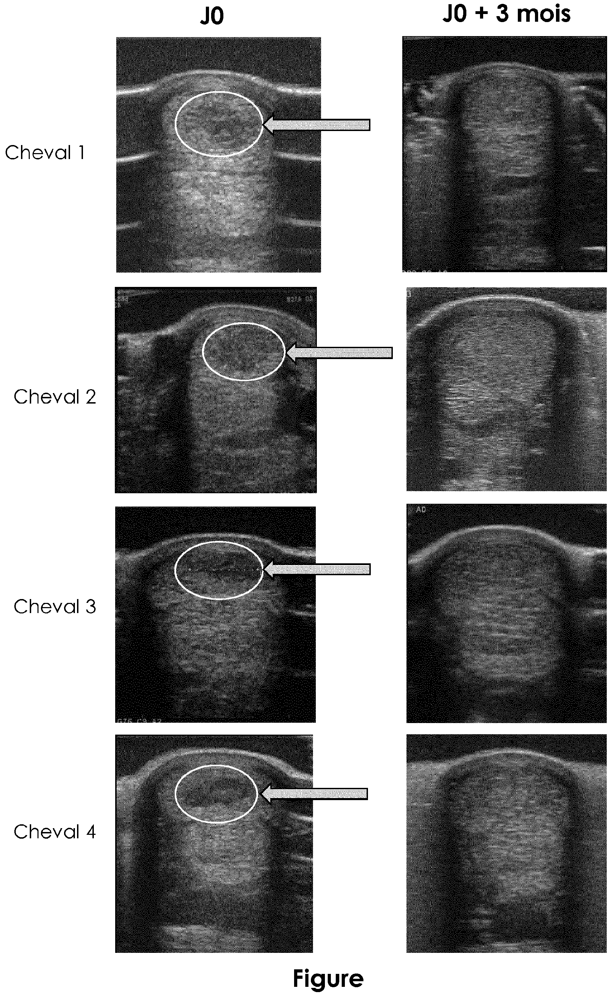

La Figure 1 représente des échographies du tendon fléchisseur superficiel de 4 chevaux souffrant d'une tendinite de ce tendon avant l'injection d'une composition de fibroblastes gingivaux (J0) puis 3 mois après l'injection (J0 + 3 mois).

La zone présentant la lésion tendineuse, plus sombre que son environnement, est entourée par un cercle blanc. L'injection de la composition à l'aide d'une seringue est effectuée dans la lésion (flèche). 3 mois plus tard le tendon a une apparence homogène, ce qui indique que la lésion a été traitée.

### EXEMPLES (Ces exemples ne relèvent pas du champ d'application des revendications, mais sont inclus à titre de référence uniquement.)

### Exemple 1 : traitement d'une tendinite

### Préparation des fibroblastes gingivaux en vue de l'administration

Une biopsie d'environ 50 mg de gencive est réalisée au scalpel chez un cheval (cheval 0) puis digérée enzymatiquement pour donner une suspension de fibroblastes gingivaux. Après numération de la suspension, un flasque de 75 cm² est ensemencé par 3.10⁵ fibroblastes gingivaux en milieu de culture complet (DMEM 4,5 g/L glucose + Glutamax (Gibco^{®}), sérum de veau fœtal (SVF) (Gibco^{®}) 20%, en présence d'antibiotiques/antimycotiques) et est incubé à 37°C en atmosphère à 5% de CO₂ jusqu'à 90% de confluence en changeant régulièrement le milieu de culture.

Les fibroblastes gingivaux sont alors récupérés. 5.10⁵ fibroblastes gingivaux sont utilisés pour ensemencer 25 mL de milieu complet en flasque de 150 cm². Une incubation est ensuite réalisé à 37°C sous une atmosphère à 5% de CO₂ en changeant régulièrement le milieu de culture, jusqu'à atteindre 90% de confluence. Ceci forme le premier passage.

2 autres passages sont réalisés. Lors du 3^{ème} passage, environ 20.10⁶ fibroblastes gingivaux sont récupérés, centrifugés, lavés dans 10 mL de milieu DMEM 0% SVF sans antibiotiques/antimycotiques, centrifugés et repris dans un volume d'environ 3 mL. Les fibroblastes gingivaux sont alors aspirés dans la seringue d'administration et conservés à 4°C jusqu'à l'administration.

### Administration des fibroblastes gingivaux

4 chevaux (chevaux 1, 2, 3 et 4) présentant une tendinite d'un tendon fléchisseur superficiel, éventuellement associée à une boiterie, ont reçu une injection de fibroblastes gingivaux hétérologues préparés comme indiqué ci-dessus. La zone lésée du tendon a été identifiée par échographie (voir la **Figure**). Elle apparait comme une zone plus dense (sombre) par rapport à son environnement.

L'injection d'environ 20.10⁶ fibroblastes gingivaux est réalisée directement dans la lésion par échoguidage (injection intra-lésionnelle).

### Résultats

3 mois après l'injection une observation échographique de la zone traitée est réalisée (voir la **Figure**). Il n'y a plus de lésion tendineuse apparente : la zone traitée apparait de manière homogène à l'échographie. La locomotion des animaux est normale.

### Exemple 2 : traitement de l'ostéochondrose

Des fibroblastes gingivaux préparés comme décrit dans l'**Exemple 1** sont injectés directement dans l'articulation du boulet d'un cheval (cheval 5) présentant une ostéochondrose du boulet associée à une boiterie.

On observe une absence de boiterie environ une semaine après l'injection.

## Revendications

1. Composition comprenant des fibroblastes gingivaux pour une utilisation dans la prévention ou le traitement d'une affection de l'appareil locomoteur chez un chien sélectionnée dans le groupe constitué d'une tendinite, d'une ostéochondrose et d'une arthrose.

2. Composition pour une utilisation selon la revendication 1, dans la prévention ou le traitement d'une tendinite d'un tendon fléchisseur, notamment superficiel ou profond des phalanges, ou d'un tendon extenseur, notamment latéral des phalanges ou antérieur des phalanges.

3. Composition pour une utilisation selon la revendication 1 ou 2, dans laquelle la composition est issue de la mise en culture d'un prélèvement de gencive.

4. Composition pour une utilisation selon l'une des revendications 1 à 3, dans laquelle la composition comprend de 5 millions à 40 millions de fibroblastes gingivaux.

5. Composition pour une utilisation selon l'une des revendications 1 à 4, dans laquelle les fibroblastes gingivaux sont hétérologues, c'est-à-dire qu'ils proviennent d'un autre chien que celui chez lequel on utilise la composition.

6. Composition pour une utilisation selon l'une des revendications 1 à 4, dans laquelle les fibroblastes gingivaux sont autologues, c'est-à-dire qu'ils proviennent du même chien que celui chez lequel on utilise la composition.

## Patentansprüche

1. Zahnfleischfibroblasten enthaltende Zubereitung für einen Gebrauch zur Vorbeugung oder Behandlung einer Erkrankung des Bewegungsapparates bei einem Hund, gewählt aus der Gruppe, bestehend aus Sehnenscheidenentzündung, Osteochondrose und Arthrose.

2. Zubereitung für einen Gebrauch nach Patentanspruch 1 in der Vorbeugung oder Behandlung einer Sehnenscheidenentzündung einer Beugesehne, insbesondere einer oberflächlichen oder tiefen der Phalangen, oder einer Strecksehne, insbesondere einer lateralen der Phalangen oder anterioren der Phalangen.

3. Zubereitung für einen Gebrauch nach einem der Patentansprüche 1 oder 2, wobei die Zubereitung aus einer Kultur einer Zahnfleischprobe hervorgegangen ist.

4. Zubereitung für einen Gebrauch nach einem der Patentansprüche 1 bis 3, wobei die Zubereitung 5 Millionen bis 40 Millionen Zahnfleischfibroblasten umfasst.

5. Zubereitung für einen Gebrauch nach einem der Patentansprüche 1 bis 4, in der die Zahnfleischfibroblasten heterolog sind, d.h. dass sie von einem anderen Hund stammen als dem, bei dem die Zubereitung angewendet wird.

6. Zubereitung für einen Gebrauch nach einem der Patentansprüche 1 bis 4, in der die Zahnfleischfibroblasten autolog sind, d.h. dass sie von demselben Hund stammen, bei dem die Zubereitung angewendet wird.

## Claims

1. A composition comprising gingival fibroblasts for use in the prevention or treatment of a disease of the locomotor system in a dog selected in the group consisting of a tendinitis, an osteochondrosis and an arthrosis.

2. The composition for use according to claim 1, in the prevention or treatment of a tendinitis of a flexor tendon, in particular superficial or deep of the phalanges, or of an extensor tendon, in particular lateral of the phalanges or anterior of the phalanges.

3. The composition for use according to claim 1 or 2, wherein the composition is derived from the culturing of a gum sample.

4. The composition for use according to any one of claims 1 to 3, wherein the composition comprises from 5 million to 40 million gingival fibroblasts.

5. The composition for use according to any one of claims 1 to 4, wherein the gingival fibroblasts are heterologous, which means they are taken from another dog than the one in whom the composition is used.

6. The composition for use according to any one of claims 1 to 4, wherein the gingival fibroblasts are autologous, which means they are taken from the same dog than the one in whom the composition is used.
